# EUROPEAN PATENT SPECIFICATION

(11) **EP 4 351 445 B1**
(45) Date of publication and mention of the grant of the patent: **14.05.2025**
(21) Application number: 22734045.2
(22) Date of filing: 10.06.2022
(51) Int. Cl.: A61B 17/16

(54) **HANDPIECE FOR SUPPORTING AND POSITIONING A SURGICAL TOOL**
HANDSTÜCK ZUM HALTEN UND POSITIONIEREN EINES CHIRURGISCHEN WERKZEUGS
PIÈCE À MAIN POUR SUPPORTER ET POSITIONNER UN OUTIL CHIRURGICAL

(30) Priority: 10.06.2021 IT 202100003062 U; 29.06.2021 IT 202100017099
(43) Date of publication of application: 17.04.2024
(73) Proprietor: Barmettler, Raffaele Franco, 20125 Milano (MI) (IT); Cavicchioli, Mirco, 41033 Concordia sulla Secchia (MO) (IT)
(72) Inventor: Barmettler, Raffaele Franco, 20125 Milano (MI) (IT); Cavicchioli, Mirco, 41033 Concordia sulla Secchia (MO) (IT)
(74) Representative: Metroconsult Srl
(86) International application number: PCT/IB2022/055402
(87) International publication number: WO 2022/259213

(56) References cited:
- WO-A2-2016/138443
- CH-A5- 693 980
- US-A1- 2013 303 330

## Description

### FIELD OF APPLICATION

The present invention relates to a handpiece or, more precisely, a spindle, for supporting and positioning a surgical tool, which may find application, for example, without however being limited thereto, in the field of surgical instruments for osteotomies.

### BACKGROUND ART

It is known that during some surgical operations it is necessary to work on teeth or bones by using a suitable cutter. Some examples of such operations include treatment of hallux valgus, dental fillings, or other mini-invasive and non-mini-invasive operations.

Reference will be made below to mini-invasive techniques that have so far required the use of specifically designed equipment, which equipment is however also used for other operations.

The so-called "mini-invasive" operations ensure as little invasiveness as possible and require a specific technique, also identified as mini-invasive or percutaneous; according to said technique, a small cutaneous incision is made, through which the surgical tools necessary for the actual operation are inserted.

In this context, surgical tools are known, such as "mini-cutters", or other instruments that may be useful or necessary from time to time, by means of which osteotomies are carried out in order to obtain correct bone realignment or to remove bone growths from incorrect areas, or caries, or the like.

Said tools are normally supported by a spindle, or handpiece, which can be manipulated, or guided, by a surgeon's hand to properly control and conduct the tool in the working area.

Said handpiece may have different shapes and, in general terms, includes a through shaft that receives motion from motor means directly connected to the handpiece; the handpiece transmits the motion to the tool while at the same time positioning it relative to itself.

For example, a handpiece may have an axial shape or may have an angle ranging from a few degrees to 90 degrees.

The tool is normally so shaped as to expel the osteotomy or caries products, such as humus, blood and debris, thus keeping the working area as clean as possible.

For simplicity's sake, the term osteotomy will hereafter refer to all cases wherein handpieces are or can be used.

In particular, due to the conformation of the tool, humus, blood and debris can easily flow up the rotation shaft and, at least partly, enter the handpiece, where they gather in one or more spaces. Such products may foul the handpiece and the internals thereof, and may become, if not removed, a possible source of infection.

The handpieces known in the art are normally made of stainless steel or other medical materials to ensure safe cleaning.

Although such handpieces can be cleaned externally, generally their interior, where pathogenic agents may reside, cannot be easily subjected to in-depth cleaning.

In order to clean them internally, in fact, it is necessary to disassemble the various components and carry out a complex and thorough cleaning, as well as a deep disinfection of each component, followed by proper lubrication. Such operations are necessary to minimize the permanence of bacteria, viruses or the like, which may otherwise first proliferate and then move into a patient's body during the next operation.

Handpieces are also known which are somehow prearranged for being quite easily cleaned; such units can be rather easily fully or partially disassembled for cleaning and disinfecting the interior and all components from anything that may have entered and/or accumulated therein.

However, the activities of disassembling, cleaning, sanitizing, lubricating and reassembling the components, if carried out properly, are time-consuming, and this prevents immediate reuse.

For this reason, and in order to ensure patient's safety, modern handpieces require considerable costs, adding to the supply cost, for cleaning and management, provided that in-depth cleaning is possible and easy.

For these very reasons, moreover, the handpiece must be replaced with a clean one before every operation, because cleaning cannot be done locally.

This requires the availability of some stock of clean handpieces in excess of those that are strictly necessary for the operations to be carried out.

In fact, as the handpieces are used during a working day, they must be replaced to avoid losing time. As a consequence, the necessary stock and the necessary cleaning operations cause organization problems not related to the surgery, as well as management and cleaning cost problems. Such costs and times affect each surgical operation to a non-negligible extent.

As a matter of fact, the mechanical components of the handpieces known in the art are typically made of metal, and are complex and articulated to provide the characteristics that the handpiece must have in order to be properly controllable and have a long service life, such long service life being necessary to amortize the supply and management costs, while avoiding that even minimal dirt residues might contaminate the next operation.

A need has therefore arisen for replacing the known handpieces with other handpieces that, while still being safe and ensuring reliable operation, can be considered as disposable units.

In particular, it is also one object of the invention to provide a handpiece that allows reducing the times and costs incurred for essential cleaning and reassembly, if a handpiece that can be reused only a few times is required.

It is another object of the present invention to provide a handpiece that can be reused for a small number of surgical operations.

It is another object of the present invention to provide handpieces that can be cleaned easily and quickly.

Thus, the cost of a handpiece according to the invention is very low, and surgical operations cost much less as well. Furthermore, organization is simplified and additional costs are eliminated, while still fulfilling all the needs that may arise during a surgical operation.

In order to overcome the drawbacks of the prior art and attain these and other objects and advantages, the Applicants have studied, tested and realized the present invention.

Document WO 2016/138443 A2 describes a surgical instrument with an articulated region.

Document CH 693 980 A5 describes a medical instrument for bone surgery.

### SUMMARY OF THE INVENTION

The present invention is set out and characterized in the independent claim. Dependent claims set out other features of the present invention or variants of the main inventive idea.

In accordance with the above-mentioned objects, a handpiece for supporting and positioning a surgical tool according to the present invention, made of plastic material or the like, is provided which can be used only once or only a few times after having been subjected to a cleaning operation, and then disposed of or subjected to a process of regeneration or recovery of its constituent material.

In accordance with one aspect of the present invention, the plastic material that constitutes said handpiece is of the eco-compatible type and meets the requirements of biocompatibility with the human body.

In accordance with a first embodiment, all of said plastic material is advantageously comprised in a single category of eco-compatible plastic material.

Advantageously, the plastic material is recyclable and the handpiece can be regenerated, or recycled, without having to be disassembled first.

According to the invention, the handpiece may have an axial longitudinal development.

According to one variant, the handpiece may have a partial axial development and then extend at a desired angle.

According to the invention, the handpiece comprises an external body and motion transmission means disposed inside said external body; said transmission means being configured to receive motion from a motor member and transmit it directly to the tool.

According to a further variant, in the case of a handpiece with an angled output, motion transmission means in angular form are provided, said means being gears or gearing means or rotating hinges or angled hinges, or other means commonly known for transmitting angular motion between two mutually angled shafts.

According to one variant of the invention, the external body and the motion transmission means, as well as spring means, bushings, abutments, etc., are all advantageously made of plastic material.

According to a further variant, the tool is advantageously of the type that can be so applied that it can be disconnected from the handpiece body as needed.

According to a further variant, the handpiece is made as two pieces, wherein the rear part is used for receiving motion and for gripping the handpiece with the hand, while the front part has an extension that may be either axial or angled by a desired value.

This makes it possible to have a standard main body and a specialized part as required from time to time.

According to the invention, the handpiece can be replaced, even during the surgical operation, and directly or indirectly applied to the means supplying rotation thereto, i.e. a direct or remote motor member.

It is within the principles of the invention that, if the connections between the handpiece and the motor member are not standardized ones, the handpiece may be fit for different connections, or that mutual and intermediate connection means, e.g. adapters, can be applied thereto and replaced.

According to the invention, said motion transmission means comprise a transmission shaft, or possibly also gearing or hinging means o other angular motion transmission means having a first end configured to be connected to the motor member and a second end configured to be connected to the tool.

According to one variant, the transmission means are associated with elastic means, e.g. a spring, which ensure a proper and stable axial connection of the same.

The handpiece further comprises means for supporting and positioning the transmission shaft, which allow the latter to rotate correctly about a longitudinal axis.

According to the invention, the supporting and positioning means are made of plastic material; they act as a bearing, a bushing, a thrust bearing or a spring, and perform a containment or locking function.

According to one variant of the invention, said supporting and positioning means comprise also at least one internal body at least partly disposed inside the external body and configured to contain and position the transmission shaft or a part thereof.

According to one variant, the body is made up of external bodies and one or more internal bodies.

According to one variant, the external body is made as one piece.

According to one variant, the external body and/or the internal body is made as two bodies which can be coupled and made integral with each other through fixed connection means.

According to one variant of the invention, the transmission shaft is divided into two or more components including, possibly in an intermediate position, angular transmission means, and comprises a first portion and at least a second portion separated and in reciprocal torsional engagement which cooperate to transmit motion while allowing for reciprocal axial sliding.

As aforementioned, the transmission shaft may be composed of one or more parts and may include the tool, or the tool can be replaced during the operation. If the tool can be replaced during the operation, both the tool and the transmission shaft have means for mutual connection, engagement and positioning and for rotary motion transmission; one example may be a flat, polygonal, etc. seat having (at least temporary) axial retaining means.

According to one variant, the handpiece has an ergonomical shape.

According to the invention, the handpiece is provided with means for centering and positioning it relative to, and possibly for establishing an axial connection with, the motor member or the terminal part thereof.

According to the invention, in order to provide engagement with the motor member, the axial position of the transmission shaft is held by spring means that ensure the proper connection thereof, which spring means are, advantageously but not necessarily, made of plastic material.

According to one variant, the external part of the handpiece includes means for positioning the operator's hand and improving the grip thereof.

According to one possible variant, the external body is internally provided with one or more seats having different diameters, with which the supporting and positioning means, such as bearings, bushings, thrust bearings or springs, can be associated to ensure proper and controlled axial positioning of the transmission shaft, said supporting and positioning means being made of plastic material, which material is advantageously specifically suited to the function to be performed.

According to one variant, at least some of the various parts of the handpiece can be connected by mutual fastening.

According to one variant, they can be connected by means of suitable rings or clips or slides.

According to a further variant, they can be connected by mutual screwing.

According to a further variant, they can be connected by gluing.

According to one variant, the motor shaft may be of the type that can be inserted into the external body from the front.

According to one variant, the motor shaft may be of the type that can be inserted into the external body from the rear.

In accordance with a further aspect, the invention concerns a surgical instrument.

Preferably, the surgical instrument comprises a handpiece for supporting and positioning a surgical tool.

Preferably, the surgical instrument comprises a motor member.

Preferably, the motor member is coupled to said handpiece for transmitting a rotary motion to the tool supported by said handpiece.

Preferably, the surgical instrument comprises a fastening member.

Preferably, the fastening member is configured to temporarily fasten a protection element to either said motor member or said handpiece in order to cover said motor member at least partially.

Preferably, said fastening member is cup-shaped.

Preferably, said fastening member has an opening facing towards said handpiece.

Preferably, said fastening member has a through hole opposite said opening.

Preferably, a radially internal surface of said protection element is coupled to a radially external surface of said fastening member.

Preferably, the coupling between the radially internal surface of the protection element and the radially external surface of the fastening member is effected by gluing.

Preferably, the coupling between the radially internal surface of the protection element and the radially external surface of the fastening member is effected by slot-and-hook coupling.

### BRIEF DESCRIPTION OF THE DRAWINGS

These and other aspects, features and advantages of the present invention will become apparent in light of the following description of some embodiments thereof, provided by way of non-limiting example with reference to the annexed drawings, wherein:
- Figs. 1, 3, 5, 7 and 9 are longitudinal sections of different exemplary embodiments of the handpiece, or spindle, in accordance with the present invention;
- Figs. 2, 4, 6 and 8 are exploded axonometric views of the respective handpieces of Figs. 1, 3, 5 and 7;
- Fig. 10 shows an illustrative example of an angled handpiece;
- Fig. 11 shows one possible variant of the handpiece, with a tool operating at an angle of 90° relative to the handpiece body;
- Figure 12 is a view of a surgical instrument according to the present invention;
- Figures 13-17 show some details of Fig. 12 according to some embodiments of the present invention.

It must be pointed out that the expressions and terms used in the present description, as well as the annexed drawings described herein, have the sole function of properly explaining and illustrating the present invention by means of some examples of embodiment, thus having only an explanatory and non-limiting function, since the protection scope of the invention is defined in the claims.

For a better understanding, the same reference numerals have been used, wherever possible, for identifying identical items common to different figures.

It remains understood that elements and features of one embodiment may be conveniently combined or incorporated in other embodiments without requiring any further clarification.

### DESCRIPTION OF SOME EMBODIMENTS OF THE PRESENT INVENTION

With reference to the annexed drawings, a spindle, or handpiece, 10 according to the present invention is configured for supporting and positioning a surgical tool 11, for the purpose of allowing an operator to guide the latter during surgical operations.

The figures annexed hereto for illustrative purposes show some examples of various implementations of the handpiece 10 according to the present invention, so as to remark the breadth of possible geometric solutions conveyed by the inventive idea.

In the annexed drawings a handpiece 10 is shown which, according to the present invention, implements only a small part of all the possible geometric formulations of the inventive idea.

It must be pointed out that the features presented in the various illustrative drawings may be variedly associated with one another to create further embodiments, being it apparent that the solutions shown herein are merely illustrative and non-limiting ones.

The surgical tool 11 associated with the handpiece 10 is adapted to remove biological material from a patient's bone portion. For example, the tool 11 may be a so-called mini-cutter.

In particular, the tool 11 is configured to rotate about an axis of rotation X1.

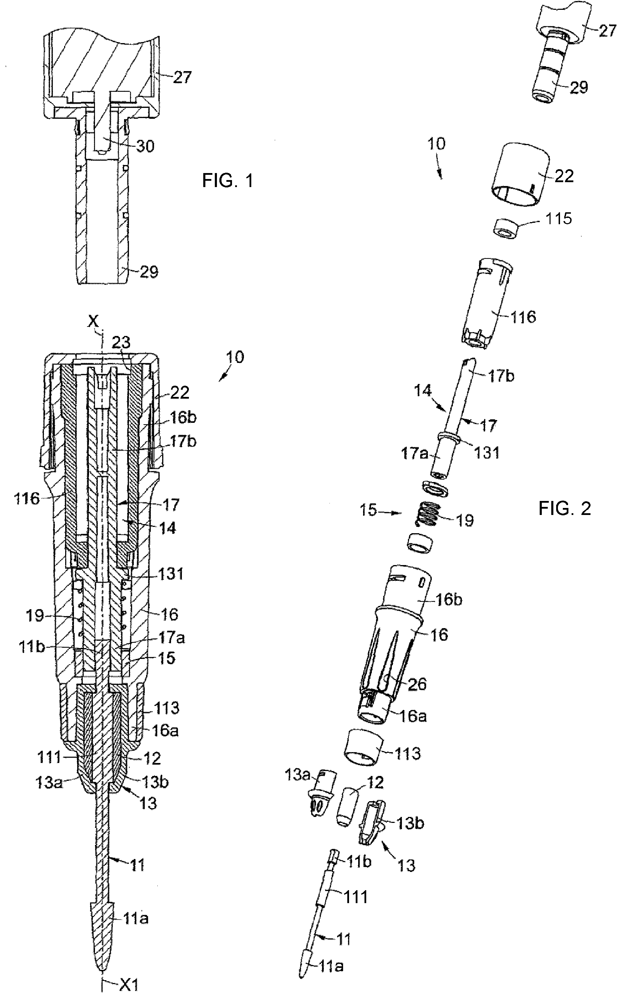

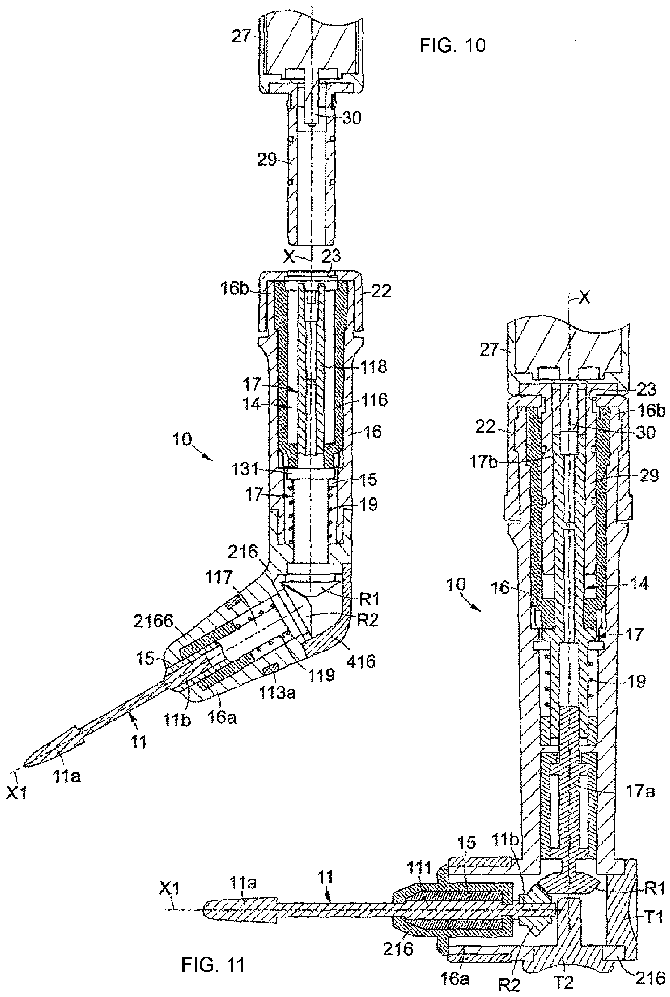

The axis of rotation X1 may be in axis with a longitudinal axis X for motion input of the handpiece 10 (Figures 1 to 9), or may be arranged at a predetermined angle, e.g. up to 90°, relative to said longitudinal axis X (Figures 10 and 11).

According to one aspect of the present invention, every component of the handpiece 10 is made of plastic material, preferably eco-compatible and, advantageously, biocompatible with the human body.

Except for the tool 11, all parts of the handpiece 10 according to the present invention can be obtained from recyclable plastic.

In some cases, e.g. for cleaning operations, also the tool 11, or part thereof 11, can be made of (reinforced) plastic material.

It is understood that the inventive idea also includes the case wherein, apart from the tool 11, the handpiece comprises some metal components that can be easily removed while the rest of the handpiece can be recycled.

The handpiece 10 comprises an elongate external body 16, suitably shaped internally and configured to be held and handled by an operator.

The external body 16 has a front end 16a from which a front portion 11a extends which belongs to the tool 11.

The external body 16 has a rear end 16b having a housing 23 configured to cooperate with a motor member 27 or with a suitable intermediate adapter.

In particular, the motor member 27 has a motor shaft 30 and also a suitable centering extension 29 that cooperates with the housing 23 to connect the motor shaft 30 axially to the handpiece 10.

Advantageously, the connection between the motor shaft 30 and the handpiece 10 preserves the sterility of the assembly.

Advantageously, the profile of the external body 16 from the rear end 16b to the front end 16a is such as to ensure an easy and steady grip while not hindering sight.

The external body 16 may have, at the rear, a fastening member 22, suitable for fastening temporary covering means, e.g. a replaceable cover or protection, on at least the motor member 27 to prevent it from getting fouled.

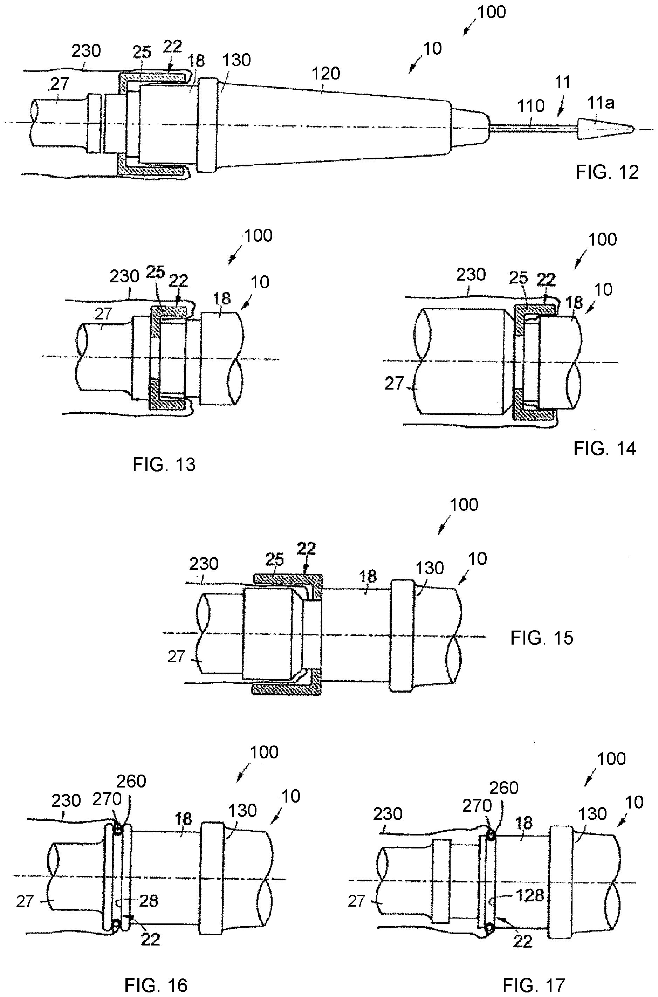

Some examples of embodiment of the fastening member 22 will be described below with reference to Figures 12-17.

The external body 16 is either made as one piece or composed of two or more elements. When two or more elements are used, the latter may be made mutually integral by gluing, snap-type fastening means, forks, pins, or the like.

By way of example, as will be made clearer later in this description:

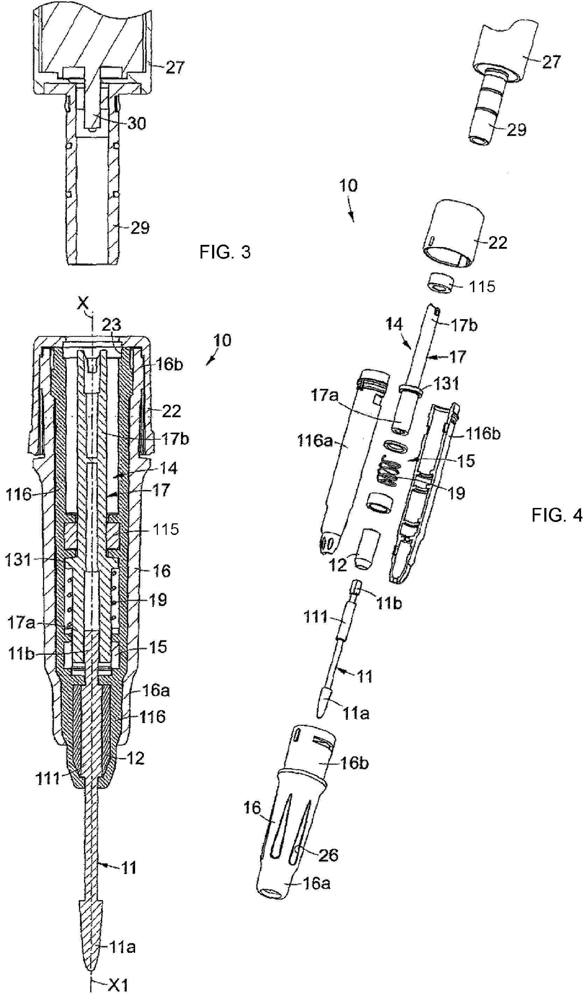

- in Figures 3 and 4 the external body 16 is formed by four elements arranged longitudinally, wherein two of such elements and the terminal part constitute the external part;
- in Figures 1 and 2, 5 and 6 the external body 16 is formed by six elements, three of which constitute the front terminal part;

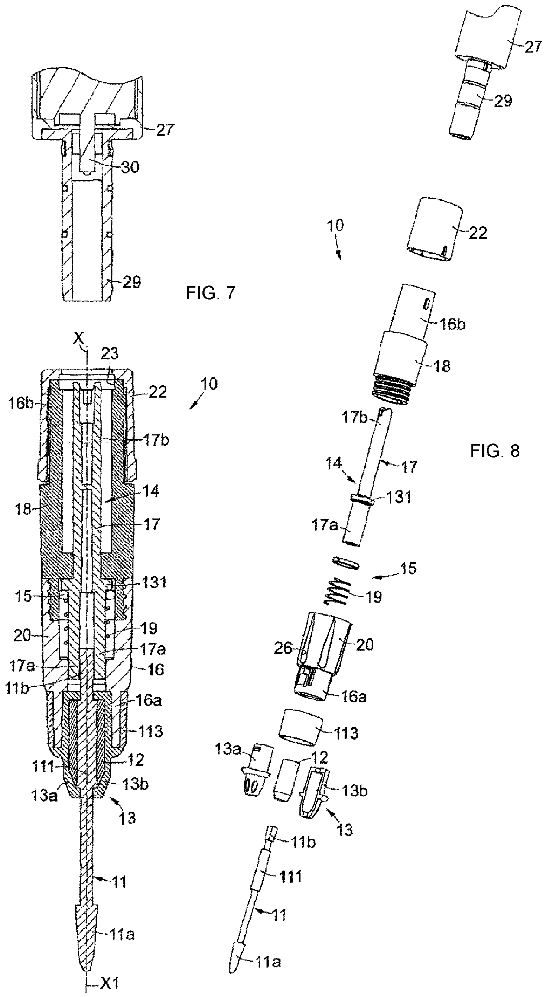

- in Figures 7 and 8 the external body 16 is formed by six elements, two of which are screwed;

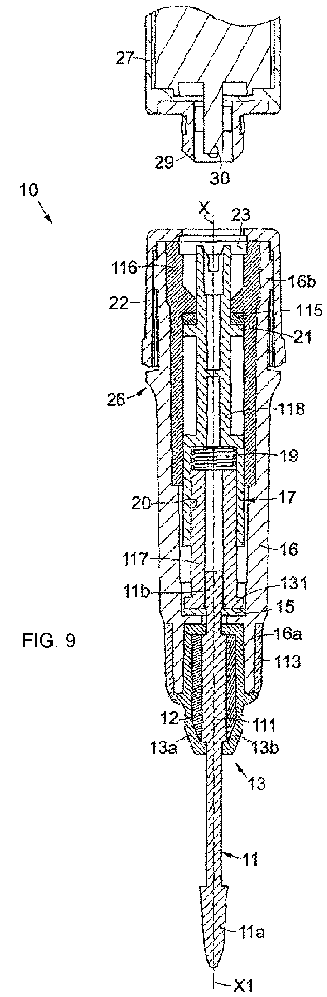

- in Figure 9 the external body 16 is formed by four elements, two of which may be made as one piece.

The external body 16 may have, on its outer surface, seats 26 for positioning the operator's hand, and may possibly have a surface adapted to improve the grip of the operator's hand.

The handpiece 10 further comprises motion transmission means 14 disposed inside the external body 16 and configured to receive motion from the motor shaft 30 and transmit it to the tool 11. The motion transmission means 14 comprise, for example, a transmission shaft 17.

The motion transmission means 14 are supported and held in position by supporting and positioning means 15.

The supporting and positioning means 15 may perform the function of bearings, or bushings, or thrust bearings, to ensure a correct axial positioning of the motion transmission means 14 with respect to the external body 16.

In particular, the transmission shaft 17 has a first end, or front end, 17a.

The first end 17a of the transmission shaft 17 cooperates, in particular through its rear portion 11b, with the tool 11 in order to transfer to the latter the rotary motion and ensure the required axial positioning thereof.

In particular, the transmission shaft 17 has a second end, or rear end, 17b.

The second end 17b of the transmission shaft 17 cooperates with the motor shaft 30, from which it receives the motion.

The transmission shaft 17 may be completely hollow in the axial direction, or it may be hollow only partially, or it may comprise a number of hollow, half-hollow and solid parts.

The supporting and positioning means 15 may be retained in seats directly formed on the external body 16, as in the embodiment shown in Figures 1 and 2, or on special auxiliary bodies.

As an alternative or in addition, the supporting and positioning means 15 comprise an internal body 116 disposed inside the external body 16, whereon one or more seats having different diameters are formed.

The internal body 116 is associated with the external body 16; the external body 16 correctly and firmly holds said internal body 116 in position after the assembly.

The type of connection between the external body 16 and the internal body 116 may be either fixed or disassemblable.

In a first embodiment thereof, the internal body 116 is made as one piece (Figures 1 and 2 and Figures 5 to 9).

According to one variant, the internal body 116 is obtained from two half-shells 116a and 116b; such half-shells 116a, 116b are coupled together along respective longitudinal profiles in order to contain and properly position the motion transmission means 14 (Figures 3 and 4).

For example, in the embodiment of Figure 3 the internal body 116 is obtained from two axial half-shells 116a, 116b (i.e. substantially separable along a plane in which the longitudinal axis X lies) that are made integral with each other and correctly positioned by the external body 16.

As an alternative or in addition, the two half-shells 116a, 116b are provided with mutual positioning and connecting means.

According to some embodiments, the mutual positioning and connecting means may be slides, hooks, screws, pins, etc., or may include a connection insert.

In the embodiments depicted in Figures 1 to 8, the transmission shaft 17 is made as one piece and has an abutment disk 131 disposed between its two ends 17a, 17b. In this case, the supporting and positioning means 15 comprise a positioning spring 19 that cooperates with the abutment disk 131. This results in the transmission shaft 17 being coupled to the motor shaft 30 and remaining in the correct position.

Preferably, the positioning spring 19 is positioned in the radially internal cavity of the external body 16.

In particular, the positioning spring 19 is separated from the internal body 116, in the axial direction, by the abutment disk 131. In other words, the positioning spring 19 and the internal body 116 are in abutment with the abutment disk 131 on axially opposite sides.

Alternatively, as in the embodiment illustrated in Figure 9, the transmission shaft 17 comprises a first shaft, or upper shaft, 117 and a second shaft, or lower shaft, 118.

Preferably, the first shaft 117 and the second shaft 118 are subject to means that define a predefined and controlled axial displacement of one or both of them.

Preferably, said means that define the mutual axial displacement are obtained in a portion of the transmission shaft 17.

Preferably, the means that define the at least partially predefined axial displacement of the upper shaft 117 and lower shaft 118 are obtained in the body of the transmission shaft 17 or, at least partly, in the internal body 116 and/or in the external body 16 of the handpiece 10.

In addition, the lower shaft 118 has a sliding seat 20 in which a part of the upper shaft 117 is contained and allowed to slide axially.

In this case, the upper shaft 117 has axial sliding means that prevent it from rotating relative to the lower shaft 118 and that cooperate with the sliding seat 20. In this case, the positioning spring 19 is disposed in the sliding seat 20, being thus interposed between the lower shaft 118 and the upper shaft 117.

Advantageously, the positioning spring 19 is also made of plastic material and may have any shape; for example, the positioning spring 19 may have a tubular shape and be made of elastic material.

The lower shaft 118, which constitutes, together with the upper shaft 117, the transmission shaft 17, has a positioning ring or abutment crown 21. Such positioning ring or abutment crown 21 is preferably disposed in a portion of the lower shaft 118 that is proximal to the connection with the motor shaft 30, i.e. located at the rear end 16b of the external body 16.

The supporting and positioning means 15 may further comprise a plastic bearing 115 whereon the abutment crown 21 can abut, e.g. as shown in Figure 9.

According to one variant, the upper shaft 117 of the transmission shaft 17, which acts upon the tool 11, may cooperate with a fixed annular support disposed in the external body 16 of the handpiece 10. Such fixed annular support stabilizes the axial position of the tool 11 relative to the handpiece 10 and allows it to rotate without difficulty.

In some embodiments, e.g. as shown in Figure 1, the tool 11 has a circumferential expansion 111 projecting in a radially external direction; at the front end 16a there is a stabilizing and gripping guide, or bushing, 12 that receives the circumferential expansion 111 of the tool 11.

Such stabilizing portion may be a bushing 12, also made of plastic material, configured to withstand lateral thrusts relative to the longitudinal axis X, reducing as a result the stress on the transmission shaft 17.

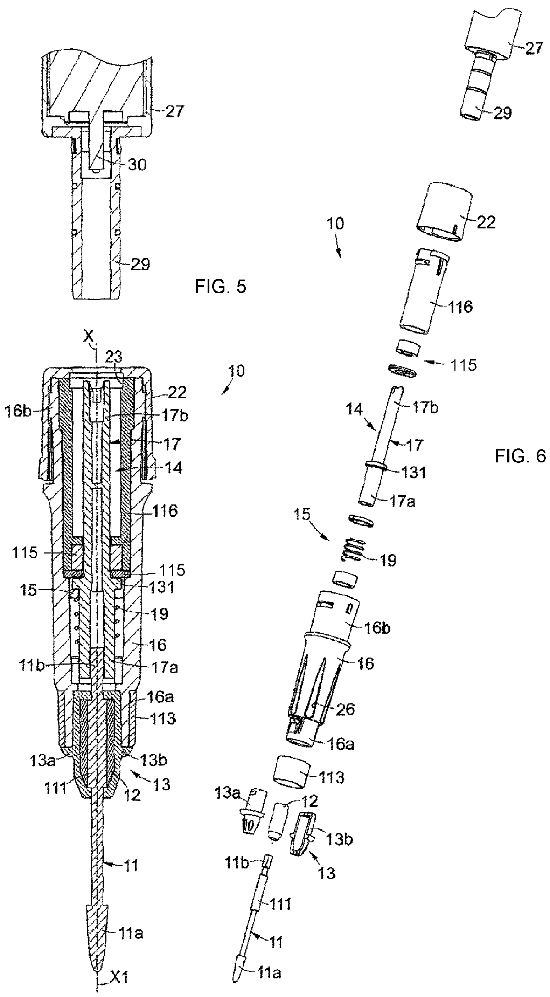

According to one variant of the invention, illustrated by way of example in Figures 1, 2 and in Figures 5-9, the bushing 12 is housed in a cover 13 that is correctly anchored axially to the external body 16.

Said cover 13 may include a capsule 113 that encloses the front portion 16a of the external body 16 to stop the dirt coming from the operation.

The cover 13 can be replaced, together with the tool 11, even while the operation is under way; the various components, e.g. the part providing angular motion, may be replaced and associated with the handpiece body in different manners according to particular technical solutions and requirements.

In Figures 1-2 and 5-9 the cover 13 is obtained from two half-shells 13a, 13b held in position relative to each other, and also relative to the handpiece 10, by the capsule 113.

According to some embodiments described herein, the handpiece 10 may have either a linear profile (Figures 1 to 9) or a non-linear profile (Figures 10 and 11).

Figure 10 illustrates, by way of example, a handpiece 10 according to the invention wherein the tool 11 lies at an angle relative to the axis of rotation of the motor shaft 30 (longitudinal axis X).

Figure 11 shows a handpiece 10 whose terminal part, which carries the tool 11, is angled by about 90° relative to the longitudinal axis X.

As aforementioned, the simplified examples shown in Figures 10 and 11 are only meant to suggest the wide range of application of the inventive idea. Still with reference to the simplified examples of Figures 10 and 11, the part with the tool 11 that is angled relative to the axis of the body gripped by the operator's hand may either be made as one piece with said body handled by the operator or be associable therewith.

The handpiece 10 may be constituted by a first body connectable to the motor member 27 and, for example, suitable for being properly gripped by the operator, and a second body that may have a linear or angled profile. Such two bodies can be associated with each other to form the required handpiece 10 according to needs arising from time to time.

In the possible solutions provided by the invention, the two mutually facing bodies may be equipped with mutual positioning and connecting means which may consist of slides, hooks, bayonets, pins, threads, or means cooperating with a connection insert or snap-type positioning means, or interference fitting means.

Figures 10 and 11 show an example of embodiment that is merely illustrative, both because that type of handpiece 10 may have different angles and because motion transmission may occur through tapered gears R1, R2, a differential gear, angular joints, flexible or spiral shafts made of plastic material, cardan joints, elastic connection joints, etc.

In the case of the angled handpiece 10 (Figure 10), it may be made up of two bodies coupled together by screwing means or by gluing, wherein both bodies can be replaced with other bodies having different characteristics.

In this variant, a spring 119 performs the function of keeping the gears R1 and R2, or any other angular transmission members as previously mentioned by way of example, in mutual rotational connection.

Still by way of example, the terminal part of the handpiece 10 may be obtained from two bodies 216 and 2166, which can then be made firmly integral with each other, e.g. by means of glue or a ring 113a.

As a possible exemplary variant for properly controlling the gears R1 and R2 transmitting the angular motion, a cover 416 may be provided, which may be either removable or firmly attached or locked.

In the variant of Figure 11, instead, in the region where the axis of rotation X1 crosses the longitudinal axis X there are two selectively removable plugs T1 and T2 which allow access to the inside of the handpiece 10 for working on the gears R1, R2 and/or on the motion transmission means 14 associated therewith.

According to a further variant of the invention, either the body connected to the motor member 27 or the applicable specialized one may be of a standard type if it is absolutely certain that no humus can enter, which will depend on their physical conformation and on the possibility or not of receiving the humus of the operation.

According to one variant, in some cases the springs 19, 119, if present, are made of metal, but according to the invention they are preferably made of plastic material.

Figures 12-17 show a surgical instrument, designated as a whole by reference numeral 100, to which the above-described handpiece 10 may belong. It should be noted, however, that the surgical instrument 100 may comprise a handpiece which is different from those described above with reference to Figures 1-11.

In addition to the handpiece 10, the surgical instrument 100 comprises a motor member 27.

The handpiece 10 may be of a replaceable type.

The handpiece 10 is adapted to be associated with the motor member 27.

The handpiece 10 has a grip zone 120 (Fig. 12) suitable to ensure proper grip by the user; in one embodiment, the grip zone 120 has a rear abutment 130 for the operator's hand.

The grip zone 120 may, for example, be made like the external body 16 described with reference to Figures 1-11.

The handpiece 10 is configured to be connected to a tool 11.

In particular, the handpiece 10 is configured to support the tool 11 at its front part. Such front part may be the front end 16a of the external body 16.

The tool 11 may be of a replaceable type.

The tool 11 comprises a head or front portion 11a, e.g. a cutting element suitable for a specific purpose ("mini-cutter"), and a stem 110 for fastening it to the handpiece 10.

The rear part of the handpiece 10 may include a connecting body 18 configured to be connected to the motor member 27.

The connecting body 18 may correspond, for example, to the rear end 16b of the external body 16.

As mentioned above, the surgical instrument 100 comprises a fastening member 22 for fastening a protection element 230. The protection element 230 is configured to cover, at least partially, the motor member 27 and possibly a cable (not shown) upstream of the motor member 27.

The fastening member 22 is configured to hold in position (preferably in a removable manner) the protection element 230 by utilizing one or more of interference, friction, elastic force, glue, adhesive material, etc.

In some embodiments, the fastening member 22 may comprise an annular element that surrounds the protection element 230 and at least one of the connecting body 18 of the handpiece 10 and the front part of the motor member 27.

In some embodiments, the fastening member 22 may comprise a fork-shaped element that elastically clamps the front terminal part of the protection element 230 on either the connecting body 18 of the handpiece 10 or the front part of the motor member 27.

In some embodiments, the fastening member may comprise elastic means that permit inserting the handpiece 10 or the motor member 27 and will then stay anchored by elasticity.

In some embodiments, the fastening member 22 may comprise means which require overlapping the ends of the front part of the protection element 230 and holding them in position through means for temporary, mutual and stable connection.

The protection element 230 may be made of any suitable material, such as, for example, a plastic or paper sheet or the like, nonwoven fabric, a tubular plastic or paper element, or the like. In some embodiments, the protection element 230 may be made from light cloth.

Preferably, the fastening member 22 can be applied easily and works by axial or orthogonal interference (elastic fork or elastic means or temporary retaining means), and can also be easily cleaned, if necessary.

In Figures 12-14, the fastening member 22 is shaped as a cup facing towards the front part of the handpiece 10.

The cup-shaped fastening member 22 can be fitted onto the connecting body 18, which may correspond to the rear end 16b of the external body 16.

In some embodiments, the fastening member 22 has, on a radially external surface thereof, first hooking portions consisting of hooks or slots; the protection element 230 is provided, in the part thereof to be anchored to the fastening member 22, with second hooking portions, consisting of slots or hooks. Thus, the constraint between the fastening member 22 and the protection element 230 is one of the slot-and-hook type. For example, tapes commercially available under the name of Velcro^{®} may be used. In addition or as an alternative, the constraint between the fastening member 22 and the protection element 230 may be attained by using an adhesive material or a glue. The fastening member 22 may be constrained to the remaining part of the handpiece (e.g. the external body 16) by interference, gluing or any other suitable mechanical coupling. Preferably, the fastening member 22 is cup-shaped, with the opening facing towards the front part of the handpiece 10. Preferably, the fastening member 22 has a through hole, on the side opposite the opening, through which the output shaft of the motor member 27 (or the adapter connected thereto) can pass.

In some embodiments, the fastening member 22 comprises an elastic element 25 configured to cooperate with either the handpiece 10 or the motor member 27 to fasten the protection element 230 by interference. In some embodiments, the elastic element 25 located within a fold of the protection element 230 may be replaced with a fork that clamps the protection element 230 from the outside.

Instead of the elastic element 25, a thread may be provided which has to be tied or stretched in order to clasp the final part of the cover onto the handpiece 10 or the motor member 27.

In some embodiments, the fastening member 22 may comprise binding means, e.g. consisting of an eyelet 260 (Figures 16 and 17) adapted to contain a cord or an elastic element 270 extending annularly, which can be fastened by means of a knot or mechanical means to the handpiece 10, whether in a cavity of the motor member 27 or in a suitable seat equipped with retaining means.

In one embodiment, the fastening means 22 may comprise a circumferential seat 28 (Fig. 16) or 128 (Fig. 17) respectively formed at the front terminal part of the motor member 27 or on the connecting body 18 (fig. 17) of the handpiece 10, so as to make it easier to firmly fasten the cord or elastic element 270.

The protection element 230 thus covers the motor member 27, and possibly also the cable upstream of the motor member 27.

When the surgical operation is complete, the protection element 230 is removed and, whenever appropriate, disposed of; the fastening member 22 can be easily and quickly cleaned and, if necessary, reused. Anyway, also the fastening member 22 may be discarded, if necessary and appropriate.

In the present invention, in addition to facilitating the application of the protection element 230, a firm and reliable (and, in some embodiments, removable) connection is maintained between the protection element 230 and the handpiece 10.

It should be noted that the fastening member 22 can also be used with handpieces having a structure different from the one shown in Figures 1-11.

The invention can thus relate to a surgical instrument 100 comprising a motor member 27 and a handpiece 10; the handpiece 10 is adapted to be associated with said motor member 27; the handpiece 10 is configured to support a tool 11, which is rotatably driven by said motor member; the surgical instrument 100 comprises a fastening member 22 for fastening, at least temporarily, a protection element 230 to either said motor member 27 or said handpiece 10 in order to cover said motor member 27 at least partially.

Preferably, said protection element 230 has a tubular shape, or is configured to form a tubular structure.

Preferably, said fastening member 22 provides a removable constraint between the protection element 230 and at least one of the motor member 27 and the handpiece 10.

Preferably, said fastening member 22 is cup-shaped or disk-shaped.

Preferably, said fastening member 22 comprises elastic means 25 configured to cooperate with either the handpiece 10 or said motor member 27.

Preferably, said fastening member 22 is configured to fasten the protection element 230 by interference with said handpiece 10.

Preferably, said fastening member 22 comprises a circumferential seat 28, 128 and binding means 26, 27 adapted to cooperate with said circumferential seat.

Preferably, said circumferential seat 28 is formed on said motor member 27.

Preferably, said circumferential seat 128 is formed on said handpiece 10.

## Claims

1. Handpiece (10), be it linear or angular, for supporting and positioning a surgical tool (11), said handpiece (10) being made of plastic material,
wherein said handpiece (10) comprises at least one external body (16) and motion transmission means (14) disposed inside said external body (16) and configured to receive motion from a motor member (27) and transmit it directly to said tool (11), said external body (16) and said motion transmission means (14) being made of plastic material,
wherein said motion transmission means (14) comprise a transmission shaft (17) having a first end (17a) configured to be connected to a motor member (27) and a second end (17b) configured to be connected to said tool (11), wherein said handpiece (10) further comprises means (15) for supporting and positioning said transmission shaft (17) and for allowing the latter to rotate about a longitudinal axis (X),
wherein said transmission shaft (17) comprises a first shaft (117) and a second shaft (118) separated and in reciprocal torsional engagement, which cooperate to transmit motion while allowing for reciprocal axial sliding,
**characterized in that**
said supporting and positioning means (15) comprise spring means (19),
wherein said spring means (19) are interposed between said first shaft (117) and said second shaft (118) of said transmission shaft (17).

2. Handpiece (10) as in claim 1, **characterized in that** said supporting and positioning means (15) further comprise bushing means (12) which at least partly surround said transmission shaft (17), said bushing means (12) being made of plastic material.

3. Handpiece (10) as in claim 1 or 2, **characterized in that** said supporting and positioning means (15) further comprise bearing means which at least partly surround said transmission shaft (17), said bearing means being made of plastic material.

4. Handpiece (10) as in any one of the preceding claims, **characterized in that** said supporting and positioning means (15) comprise an internal body (116) at least partly disposed inside said external body (16) and configured to position said transmission shaft (17).

5. Handpiece (10) as in claim 4, **characterized in that** said internal body (116) is obtained from two longitudinal half-shells (116a, 116b) made integral with each other and correctly positioned by said external body (16).

6. Handpiece (10) as in any one of the preceding claims, **characterized in that** said external body (16) comprises a housing (23) configured to cooperate with a motor shaft (30) of said motor member (27), and **in that** holding means are provided in said housing (23), which are configured to permit the momentary presence of temporary means for covering said motor member (27).

7. Handpiece (10) as in any one of the preceding claims, **characterized in that** said plastic material is the same or is substantially comprised in the same class of products for all components of said handpiece (10), such as the external body (16), the motion transmission means (14), and the supporting and positioning means (15).

8. Surgical instrument (100), comprising:
a handpiece (10) in accordance with any one of the preceding claims;
a motor member (27), coupled to said handpiece (10) for transmitting a rotary motion to the tool (11) supported by said handpiece (10).

9. Surgical instrument (100) as in claim 8, further comprising a fastening member (22) for temporarily fastening a protection element (230) to either said motor member (27) or said handpiece (10) in order to cover said motor member (27) at least partially.

10. Surgical instrument (100) as in claim 9, wherein said fastening member (22) is cup-shaped, with the opening facing towards said handpiece (10) and with a through hole opposite said opening.

11. Surgical instrument (100) as in claim 10, wherein a radially internal surface of said protection element (230) is coupled to a radially external surface of said fastening member (22).

12. Surgical instrument as in claim 11, wherein the coupling between the radially internal surface of the protection element (230) and the radially external surface of the fastening member (22) is effected by one of:
gluing;
slot-and-hook coupling;
adhesive material.

## Patentansprüche

1. Lineares oder winkelförmiges Griff (10) zum Halten und Positionieren eines chirurgischen Werkzeugs (11), wobei der Griff (10) aus Kunststoff hergestellt ist,
wobei der Griff (10) mindestens einen Außenkörper (16) und ein Bewegungsübertragungsmittel (14) umfasst, das innerhalb des Außenkörpers (16) angeordnet ist und so konfiguriert ist, dass es eine Bewegung von einem Motorelement (27) aufnimmt und sie direkt auf das Werkzeug (11) überträgt, wobei der Außenkörper (16) und das Bewegungsübertragungsmittel (14) aus Kunststoffmaterial hergestellt sind,
wobei die Bewegungsübertragungsmittel (14) eine Übertragungswelle (17) mit einem ersten Ende (17a), das so konfiguriert ist, dass es mit einem Motorelement (27) verbunden werden kann, und einem zweiten Ende (17b), das so konfiguriert ist, dass es mit dem Werkzeug (11) verbunden werden kann, umfassen, wobei der Griff (10) ferner Mittel (15) zum Tragen und Positionieren der Übertragungswelle (17) und zum Ermöglichen, dass sich letztere um eine Längsachse (X) dreht, umfasst,
wobei die Übertragungswelle (17) eine erste Welle (117) und eine zweite Welle (118) umfasst, die voneinander getrennt sind und in gegenseitigem Dreheingriff stehen, die zusammenwirken, um Bewegung zu übertragen, während sie ein gegenseitiges axiales Gleiten ermöglichen,
**dadurch gekennzeichnet, dass**
die Stütz- und Positionierungsmittel (15) Federmittel (19) umfassen,
wobei die Federmittel (19) zwischen der ersten Welle (117) und der zweiten Welle (118) der Übertragungswelle (17) angeordnet sind.

2. Griff (10) nach Anspruch 1, **dadurch gekennzeichnet, dass** die Stütz- und Positioniermittel (15) ferner Buchsenmittel (12) umfassen, die die Übertragungswelle (17) zumindest teilweise umgeben, wobei die Buchsenmittel (12) aus Kunststoffmaterial hergestellt sind.

3. Griff (10) nach Anspruch 1 oder 2, **dadurch gekennzeichnet, dass** die Stütz- und Positioniermittel (15) ferner Lagermittel umfassen, die die Übertragungswelle (17) zumindest teilweise umgeben, wobei die Lagermittel aus Kunststoffmaterial hergestellt sind.

4. Griff (10) nach einem der vorhergehenden Ansprüche, **dadurch gekennzeichnet, dass** die Stütz- und Positioniermittel (15) einen inneren Körper (116) umfassen, der zumindest teilweise innerhalb des äußeren Körpers (16) angeordnet und so konfiguriert ist, dass er die Übertragungswelle (17) positioniert.

5. Griff (10) nach Anspruch 4, **dadurch gekennzeichnet, dass** der Innenkörper (116) aus zwei länglichen Halbschalen (116a, 116b) besteht, die fest miteinander verbunden und durch den Außenkörper (16) korrekt positioniert sind.

6. Griff (10) nach einem der vorhergehenden Ansprüche, **dadurch gekennzeichnet, dass** der Außenkörper (16) ein Gehäuse (23) umfasst, das so gestaltet ist, dass es mit einer Motorwelle (30) des Motorteils (27) zusammenwirkt, und dass in dem Gehäuse (23) Haltemittel vorgesehen sind, die so gestaltet sind, dass sie das vorübergehende Vorhandensein von Mitteln zur Abdeckung des Motorteils (27) ermöglichen.

7. Griff (10) nach einem der vorhergehenden Ansprüche, **dadurch gekennzeichnet, daß** das Kunststoffmaterial für alle Komponenten des Griffes (10), wie z.B. den äußeren Körper (16), das Bewegungsübertragungsmittel (14) und das Stütz- und Positionierungsmittel (15), das gleiche ist oder im wesentlichen aus der gleichen Produktklasse besteht.

8. Chirurgisches Instrument (100), bestehend aus:
ein Griff (10) nach einem der vorhergehenden Ansprüche;
ein Motorelement (27), das mit dem Griff (10) gekoppelt ist, um eine Drehbewegung auf das von dem Griff (10) getragene Werkzeug (11) zu übertragen.

9. Chirurgisches Instrument (100) nach Anspruch 8, ferner umfassend ein Befestigungselement (22) zum vorübergehenden Befestigen eines Schutzelements (230) entweder an dem Motorteil (27) oder dem Griff (10), um das Motorteil (27) zumindest teilweise abzudecken.

10. Chirurgisches Instrument (100) nach Anspruch 9, wobei das Befestigungselement (22) schalenförmig ist, wobei die Öffnung dem Griff (10) zugewandt ist und mit einem Durchgangsloch gegenüber der Öffnung.

11. Chirurgisches Instrument (100) nach Anspruch 10, wobei eine radial innere Oberfläche des Schutzelements (230) mit einer radial äußeren Oberfläche des Befestigungselements (22) verbunden ist.

12. Chirurgisches Instrument nach Anspruch 11, wobei die Kopplung zwischen der radial inneren Oberfläche des Schutzelements (230) und der radial äußeren Oberfläche des Befestigungselements (22) durch eine der folgenden Maßnahmen bewirkt wird:
Kleben;
Schlitz-Haken-Kupplung;
Klebstoff.

## Revendications

1. Manche (10), linéaire ou angulaire, destiné à supporter et à positionner un outil chirurgical (11), ledit manche (10) étant en matière plastique,
dans lequel ledit manche (10) comprend au moins un corps externe (16) et un moyen de transmission de mouvement (14) disposé à l'intérieur dudit corps externe (16) et configuré pour recevoir le mouvement d'un élément moteur (27) et le transmettre directement audit outil (11), ledit corps externe (16) et ledit moyen de transmission de mouvement (14) étant en matière plastique,
dans lequel ledit moyens de transmission de mouvement (14) comprend un arbre de transmission (17) ayant une première extrémité (17a) configurée pour être reliée à un élément moteur (27) et une deuxième extrémité (17b) configurée pour être reliée audit outil (11), dans lequel ledit manche (10) comprend en outre des moyens (15) pour supporter et positionner ledit arbre de transmission (17) et pour permettre à ce dernier de tourner autour d'un axe longitudinal (X),
dans lequel ledit arbre de transmission (17) comprend un premier arbre (117) et un second arbre (118) séparés et en prise de torsion réciproque, qui coopèrent pour transmettre le mouvement tout en permettant un glissement axial réciproque,
**caractérisé par le fait que**
lesdits moyens de support et de positionnement (15) comprennent des ressorts (19),
dans lequel lesdits moyens à ressort (19) sont interposés entre ledit premier arbre (117) et ledit second arbre (118) de l'arbre de transmission (17).

2. Manche (10) selon la revendication 1, **caractérisé en ce que** lesdits moyens de support et de positionnement (15) comprennent en outre des moyens de bague (12) qui entourent au moins partiellement ledit arbre de transmission (17), lesdits moyens de bague (12) étant en matière plastique.

3. Manche (10) selon la revendication 1 ou 2, **caractérisé en ce que** lesdits moyens de support et de positionnement (15) comprennent en outre des paliers qui entourent au moins partiellement ledit arbre de transmission (17), lesdits paliers étant en matière plastique.

4. Manche (10) selon l'une quelconque des revendications précédentes, **caractérisé en ce que** lesdits moyens de support et de positionnement (15) comprennent un corps interne (116) disposé au moins en partie à l'intérieur dudit corps externe (16) et configuré pour positionner ledit arbre de transmission (17).

5. Manche (10) selon la revendication 4, **caractérisé en ce que** ledit corps interne (116) est obtenu à partir de deux demi-coquilles longitudinales (116a, 116b) rendues solidaires l'une de l'autre et correctement positionnées par ledit corps externe (16).

6. Manche (10) selon l'une quelconque des revendications précédentes, **caractérisé en ce que** ledit corps externe (16) comprend un logement (23) configuré pour coopérer avec un arbre moteur (30) dudit organe moteur (27), et **en ce que** des moyens de maintien sont prévus dans ledit logement (23), qui sont configurés pour permettre la présence momentanée de moyens temporaires de recouvrement dudit organe moteur (27).

7. Manche (10) selon l'une quelconque des revendications précédentes, **caractérisé en ce que** ladite matière plastique est la même ou est substantiellement comprise dans la même classe de produits pour tous les composants dudit manche (10), tels que le corps externe (16), le moyen de transmission de mouvement (14), et les moyens de support et de positionnement (15).

8. Instrument chirurgical (100), comprenant :
un manche (10) selon l'une quelconque des revendications précédentes ;
un élément moteur (27), couplé à ledit manche (10) pour transmettre un mouvement de rotation à l'outil (11) supporté par ledit manche (10).

9. Instrument chirurgical (100) selon la revendication 8, comprenant en outre un élément de fixation (22) pour fixer temporairement un élément de protection (230) à l'élément moteur (27) ou au manche (10) afin de couvrir au moins partiellement l'élément moteur (27).

10. Instrument chirurgical (100) selon la revendication 9, dans lequel ledit élément de fixation (22) est en forme de coupelle, avec l'ouverture orientée vers ledit manche (10) et avec un trou traversant à l'opposé de ladite ouverture.

11. Instrument chirurgical (100) selon la revendication 10, dans lequel une surface radialement interne de l'élément de protection (230) est couplée à une surface radialement externe de l'élément de fixation (22).

12. Instrument chirurgical selon la revendication 11, dans lequel le couplage entre la surface radialement interne de l'élément de protection (230) et la surface radialement externe de l'élément de fixation (22) est effectué par l'un des éléments suivants :
collage ;
l'accouplement à fente et à crochet ;
matériau adhésif.
